# EUROPEAN PATENT SPECIFICATION

(11) **EP 4 373 538 B1**
(45) Date of publication and mention of the grant of the patent: **17.06.2026**
(21) Application number: 22747729.6
(22) Date of filing: 22.07.2022
(51) Int. Cl.: A61M 1/00, A61F 13/00, A61F 13/05, A61F 17/00

(54) **A SELECTIVELY CONFIGURABLE WOUND DRESSING**
SELEKTIV KONFIGURIERBARER WUNDVERBAND
PANSEMENT SÉLECTIVEMENT CONFIGURABLE

(30) Priority: 23.07.2021 GB 202110649
(43) Date of publication of application: 29.05.2024
(73) Proprietor: ConvaTec Limited, Deeside, Flintshire CH5 2NU (GB)
(72) Inventor: LAY, Amelia, Flintshire CH5 2NU (GB); PEERS, Andrew, Flintshire CH5 2NU (GB); GILDING, Duncan, Flintshire CH5 2NU (GB); BALLAMY, Lucy, Flintshire CH5 2NU (GB); LEYLAND, Sally, Flintshire CH5 2NU (GB); POWELL, Shauna, Flintshire CH5 2NU (GB)
(74) Representative: Wilson Gunn
(86) International application number: PCT/GB2022/051917
(87) International publication number: WO 2023/002210

(56) References cited:
- WO-A2-2020/146154
- US-A1- 2015 258 259
- US-A1- 2020 000 985

## Description

### Technical Field of the Invention

The present invention relates to a wound dressing selectively configurable for use with a pressure gradient wound therapy apparatus or for use without a pressure gradient wound therapy apparatus.

### Background to the Invention

Health care professionals (HCPs) are required to evaluate and treat a variety of wounds with different requirements which can change as treatment progresses. Accordingly, HCPs keep stocks of different types of dressings for different requirements.

Pressure gradient wound therapy (positive or negative) is one known way of treating various wound types. Typically, this involves applying a pressure differential between a sealed region of a wound dressing and te surrounding environment to assist with healing the wound, e.g. through removal of oedema, increasing blood flow, mechanical contraction of the wound, increasing formation of granulation tissue and/or active removal of excess exudate from the wound. Wound therapy of this type is particularly effective for the treatment of open traumatic, non-traumatic and chronic wounds.

Amongst different types of dressings are those designed and intended for use as an advanced wound dressing to manage exudate and protect th wound. These can, for example, have a wound contact layer including gelling fibres, such as the Hydrofiber (RTM) technology included in Aquacel (RTM) surgical dressings available from ConvaTec Ltd of Deeside, UK, which transform into a gel on contact with wound fluid. (Such wound dressings are not intended for use with pressure gradient wound therapy, nor used for such applications in practice, owing to their construction.)

Other types of wound dressing are specially adapted to be used in conjunction with a pressure gradient wound therapy apparatus, e.g. with a negative pressure wound therapy (NPWT pump). In their original incarnation, NWPT systems had large, heavy (not portable/wearable) pump arrangements connected via tubing to the wound; at the
large, heavy (not portable/wearable) pump arrangements connected via tubing to the wound; at the wound, a reticulated open cell foam dressing is introduced into the wound and a separate adhesive drape is placed over the top. To connect the tubing to the wound in these large systems, a health care professional pinches the drape and foam beneath, and snips a hole through both drape and foam to form an aperture in the top for connection to the tubing. These systems and the two-part wound dressing (i.e. separate foam and drape) are still widely used in hospitals where professional staff are on-hand to set up te systems.

More recently, and especially in portable or wearable pressure gradient wound therapy systems intended for home-use, one-piece dressings have been introduced, in which an adhesive covering layer and dressing are integrated. An example of such a dressing is the Avelle (RTM) dressing available from ConvaTec Limited of Deeside UK. That dressing has a covering film layer with an adhesive border provided around its periphery to form a seal around the wound and a wound contact layer of stitch-bonded Hydrofiber (RTM) material. A foam pressure-distribution layer is provided between the covering layer and the wound contact layer (adjacent the covering layer) and additional layers of fenestrated Hydrofiber (RTM) layers are provided between the wound contact layer and the pressure-distribution layer. The covering layer is provided with an aperture, to allow connection of tubing from the negative pressure source and in this example, the dressing includes an "airway" extending from the aperture to a connector for connection to tubing through which negative pressure is provided.

An example of a wound dressing for use in pressure gradient wound therapy systems is found in US 2015/0258259 A1 which describes a wound therapy device comprising an outer part comprising an inner part facing surface provided with an inner part facing opening, and a first connection channel to establish fluid communication between said inner part facing opening and a connecter opening adapted to be connected to an external device. The device further comprises an inner part comprising an outer part facing surface provided with an outer part facing opening, and a second connection channel to establish fluid communication between said outer part facing opening and an opening adapted to be arranged in connection with a wound, wherein said inner part is adapted to be arranged under a wound cover film in relation to the wound, and a locking means adapted to attach and lock said outer part to said inner part, having said wound cover film arranged between said parts, and such that said inner part facing opening and outer part facing opening are positioned to essentially correspond to each other.

A further example is found in WO 2020/146154 A2 which describes a releasable connector for a negative pressure wound therapy system having a drape and a tube includes a base and a tube fitting. The base is configured to be coupled to the drape such that a substantially air-tight seal is formed between the base and the drape. The base includes a base locking member.

Naturally, where a hospital or the like provides both "normal" wound dressings and those for use with pressure gradient wound therapy it needs to keep stocks of both, in various different sizes, which requires space, and lends complexity to the stock-ordering and stock selection process.

The present inventors have also identified that it would be beneficial to be able to provide a dressing that could be used for both pressure gradient wound therapy and "normal" treatment of wounds without a pressure gradient, in order that such treatment could be applied as and when required, and if no longer required, the dressings could still be used.

It is an aim of an embodiment or embodiments of the invention to overcome or at least partially mitigate one or more problems with the prior art and/or to provide an improved wound dressing.

### Summary of the Invention

According to a broad aspect of the disclosure there is provided a wound dressing selectively configurable for use in a pressure gradient wound therapy system and for use without a pressure gradient wound therapy system as defined int he appended claims.

The wound dressing comprises a wound contact layer; the wound contact layer comprises a first surface for contacting a wound and an opposing second surface; the wound dressing comprises a covering layer; the covering layer has a first surface facing the wound contact layer; the first surface defines a cavity; the covering layer comprises an opposing second surface; the second
surface of the covering layer comprises a planar base plate of a two-part port; the two-part port for connecting to a source of non-atmospheric pressure; the base plate is configured to sealingly receive a connector part of the two-part port.

The wound dressing is a one-piece dressing. That is to say, the covering layer and a body of the wound dressing are provided as an integral item, preferably including an adhesive layer and preferably including a removable release layer. The body of the wound dressing comprises the wound contact layer.

According to an aspect of the invention there is provided a one-piece wound dressing as defined in the appended claim 1.

Such a wound dressing can be used either with a pressure gradient wound therapy system, or without. Thus, stock-keeping is simplified Moreover, if, during "normal" treatment without pressure gradient it is determined that it would be beneficial for pressure gradient wound therapy to be used, such a system could be added, by configuring the wound dressing accordingly (i.e. by applying a connector part of the two-part port; and optionally cutting or opening an aperture in the cover layer of the wound dressing) and attaching a source of non-atmospheric pressure (e.g. tubing from a NWPT pump). Notably, this could be done without the need for an additional dressing change, thus reducing the risk of wound infection. Similarly, if a wound is being treated with a pressure gradient, e.g. NWPT and the amount of exudate produced is reduced, so that NWPT is no longer required, a patient would be able to use up the stock of dressings without the NWPT system, by configuring them for use without the system. Further the reduced profile of the base plate (as opposed to a full port) reduces the risk of pressure sores when the dressing is used without a pressure gradient wound therapy system.

When used herein and throughout the specification the term "pressure gradient wound therapy apparatus" is intended to cover a wound therapy apparatus wherein a pressure differential (either positive or negative) is applied between a sealed region of the wound dressing and the surrounding environment.

As used herein, negative pressure wound therapy is a therapeutic technique using a suction dressing to remove excess exudation and promote healing in acute or chronic wounds. A vacuum of -50 to -200 mm Hg, or -75 to -150 mm Hg may be applied with typical negative pressure of -80 to -130 mm Hg, -100 to -130 mm Hg, or often about -125 mm Hg being applied to a wound.

For positive pressure wound therapy, a net positive pressure is applied to the wound, which may include providing simultaneous aspiration and irrigation of the wound. Positive pressure wound therapy may be carried out at a positive pressure of up to 50% atm., typically at a low positive pressure of up to 20% atm., more usually up to 10% atm. at the wound. Positive pressure wound therapy is known and referred to in US20180140755.

Optional features set out below may apply to any aspect of the invention. The covering layer may comprise an aperture. The aperture may be configured to provide fluid communication between the wound dressing cavity and an environment external to the dressing (e.g.. a source of non-atmospheric pressure). The aperture may thus allow for use of the wound dressing with a pressure gradient would therapy system.

The base plate may comprise an aperture to allow fluid to pass through the base plate. The aperture in the base plate may be aligned with the aperture in the covering layer. The aperture in the base plate may be arranged in the centre of the planar base plate. The base plate may be annular.

The base plate may be flexible. The base plate may be thermoformed. The base plate may be comprised of thermoplastics, preferably the base plate may be comprised of thermoplastic polyurethane (TPU), or preferably the base plate may be comprised of silicone.

The base plate may be joined to the second surface of the covering layer with an adhesive. The base plate may be joined to the second surface of the covering layer by a weld.

The base plate according to the invention as claimed comprises a microbial barrier. The aperture in the covering layer may be covered with a microbial barrier. The microbial barrier may be a layer of filter material. The microbial barrier may be an occlusive material. The microbial barrier may be a mechanical valve, such as a one-way valve, to allow flow of fluid (e.g. liquid) from the first surface of the covering layer to the second surface of the covering layer, but not *vice versa.*

The base plate may comprise a means for connecting the base plate to the connector part. The base plate may comprise one or more projections for connecting the connector part to the base plate. The base plate may comprise two projections, or the base plate may comprise three projections, or the base plate may comprise three projections, or the base plate comprise four projections, or the base plate may comprise five or more projections, preferably the base plate comprises three projections. The projections may project out of the plane of the base plate. The projections may project away from the cover layer. The projections may be configured to connect to the connector part by means of a snap fit. The snap fit may be a cantilever snap fit. Each projection may comprise one or more clips to snap fit to the connector part. The connector part may comprise one or more recesses corresponding to the one or more clips. The projections may be arranged adjacent the aperture in the base plate. The clips may extend radially, for example radially outwardly. The base plate and connector part may alternatively connect by means of a screw thread, a thread being provided on one or both of the base plate (for example on the or each projection) and the connector part. The base plate and connector part may connect by means of a bayonet fitting, in which case the base plate may be provided with a slot or a pin of a bayonet fitting.

The base plate and/or the connector part may comprise a sealing element. The sealing element may be an O-ring.

The base plate may have a maximum thickness of no more than 10mm; 5mm; 3mm; or 2mm. For example the base plate may have a maximum thickness of between 5.0mm and 10mm. This range of 5.0-10mm is particularly suited to a base plate having a snap fit. In another example, the base plate may have a maximum thickness of 0.1-5mm. This range of 0.1-5mm is preferable in terms of reducing skin pressure damage risk, but may be difficult to achieve with a snap-fit connection.

The connector part defined above may be the connector part of any other aspect of this invention as outlined below and have any optional features thereof.

The base plate is preferably located out of alignment (i.e. vertical alignment in use) with the centre of the dressing. For example it may be located towards the periphery of the covering layer and/or a pressure distribution layer (or the wound when in use). Since in use in negative pressure therapy exudate is drawn through an aperture in the base plate, offsetting it assists in the spread of exudate across the full extent of the pressure distribution layer (and across an absorbent layer where an absorbent layer is provided).

A pressure distribution layer may be provided adjacent to the cover layer. The pressure distribution layer may be provided in, e.g. contained in, the cavity. The pressure distribution layer may be gas and liquid permeable and particularly moisture vapour permeable. The pressure distribution layer serves to aid access of exudate to a greater area of the absorbent layer by allowing it to spread under the distribution layer. The pressure distribution layer also serves to even out the negative pressure applied to the wound over the whole dressing (when used for NWPT). The pressure distribution layer is preferably configured to distribute exudate and negative pressure over the dressing. The pressure distribution layer is preferably a foam layer such as a polyester foam of the type XD4200AS manufactured by Caligen or another suitable reticulated foam.

An adhesive layer may be provided so as to form an adhesive border. The adhesive layer may be provided on the underside of the covering layer. The adhesive border may be provided at the periphery of the dressing arranged to adhere the dressing to the skin surrounding the wound to form a fluid tight seal. The adhesive layer may be provided with perforations to assist transport of exudate and fluid through the dressing. The adhesive layer may also be applied to any of the other layers to provide an island configuration.

The base plate may be located in a region of the covering layer within that defined by the adhesive border. For example it may be located towards the periphery of the region of the covering layer inwards of the adhesive border. In the configuration for use without a pressure gradient wound therapy system the region of the covering layer within that defined by the adhesive border may be closed, sealed and/or uninterrupted, e.g. a continuous unbroken membrane. This restricts/prevents microbes, bacteria or the like from entering the wound dressing and hence from entering the wound. In the configuration for use with a pressure gradient wound therapy system the region of the covering layer within the adhesive border may be interrupted by an opening, the opening being an aperture in the covering later for connection to a source of non-atmospheric pressure

The wound dressing may include a dressing body comprising an absorbent material for contacting the wound, i.e. which may be positioned in contact with a wound, in use. The dressing body may be formed from one of more layers. The dressing body may be configured to absorb exudate from the wound, aided by the action of a connected pump assembly. The dressing body may comprise an absorbent foam material, for example a layer of absorbent foam material. The foam material may comprise a superabsorbent material, for example a superabsorbent foam material. The dressing body may be formed of a hydrocolloid material which may gel in the presence of an exudate. The hydrocolloid material may comprise a layer or multiple layers of gelling fibres and absorbent materials. The covering layer may be constructed of a thin film layer (e.g. a polyurethane) enabling moisture vapour to exit the dressing at an increased rate. This combination is particularly suitable for allowing the wound therapy apparatus to manage fluid without the need of a canister. This may be referred to as a "canister-less" or "canister-free" system. In a variant, the wound dressing may be operable to be fluidly connected to a canister into which exudate removed from the wound may be withdrawn. The adhesive border may define an interior region of the wound dressing. The dressing body may be provided in the cavity of the wound dressing.

The wound dressing may comprise a release layer, the release layer being removable to reveal the adhesive border.

The wound dressing may have a thickness between 1mm to 20mm, or 2mm to 10mm, or 3mm to 7mm, for example.

The pressure distribution layer may be a foam layer. The wound dressing may comprise the outer covering layer, the pressure distribution layer, one or more absorbent layer(s) and a silicone gel wound contact layer. The wound dressing may comprise an outer covering layer and one or more absorbent layer(s) in combination with a gel-forming fibre. The gel-forming fibre typically is in direct contact with the wound, and thus no additional wound contact layer is required i.e., a silicone gel wound contact layer does not require a silicone gel layer.

Gel-forming fibres include hygroscopic fibres which upon the uptake of wound exudate become moist slippery or gelatinous. The gel forming fibres can be of the type which retain their structural integrity on absorption of exudate or can be of the type which lose their fibrous form and become an amorphous or structureless gel. The gel forming fibres are preferably sodium carboxymethylcellulose fibres, chemically modified cellulosic fibres, alkyl sulphonate modified cellulosic fibres such as those described in WO2012/061225, pectin fibres, alginate fibres, chitosan fibres, hyaluronic acid fibres, or other polysaccharide fibres or fibres derived from gums. The cellulosic fibres preferably have a degree of substitution of at least 0.05 carboxymethyl groups per glucose unit. The gel forming fibres preferably have an absorbency of at least 2 grams 0.9% saline solution per gram of fibre (as measured by the free swell method).

The gel forming fibres are preferably chemically modified cellulosic fibres in the form of a fabric and in particular carboxymethylated cellulose fibres as described in PCT WO00/01425 to Azko Nobel UK Ltd, and can be provided by a layer of gel forming fibres preferably located in a port of the cover layer or as a layer of fibres in a conduit of the wound dressing. When present in the conduit, the layer of fibres can also serve to keep the conduit open to the passage of fluid in the event that the conduit is kinked or otherwise restricted by being lain on or leaned on by the user. The carboxymethylated cellulosic fabrics preferably have a degree of substitution between 0.12 to 0.35 as measured by IR spectroscopy (as defined in WO00/01425) more preferably a degree of substitution of between 0.20 and 0.30 and are made by carboxymethylating a woven or non-woven cellulosic fabric such that the absorbency is increased. Particular preferred fabrics have an absorbency of between 10g/g of sodium/calcium chloride as defined above to 30g/g of sodium/calcium chloride as measured by the method described in BS EN 13726-1 (2002) "Test methods for primary wound dressings", section 3.2 "Free swell absorptive capacity". Particularly preferred fabrics have an absorbency of 15g/g to 25g/g and most preferred of 15g/g to 20g/g of sodium/calcium chloride as measured by the method defined above.

The cellulosic fabric preferably consists solely of cellulosic fibre but may contain a proportion of non-cellulosic textile fibre or gel forming fibre. The cellulosic fibre is of known kind and may comprise continuous filament yarn and/or staple fibre. The carboxymethylation is generally performed by contacting the fabric with an alkali and a carboxymethylating agent such a chloracetic acid in an aqueous system. The fabric is preferably of a non-woven type to reduce shedding in the wound on cutting the dressing. Preferably the fabric is hydroentangled and thus comprises a series of apertures on a microscopic scale.

Where present, the absorbent layer of the wound dressing is capable of absorbing exudate from the wound and allowing the passage of fluid through it. The absorbent layer can comprise any absorbent capable of absorbing exudate while allowing the passage of fluid through it, such as a foam, sponge or fibre-based material, preferably the absorbent layer is provided by gel forming fibres of the same type or of a different type as those discussed above. The gel-forming fibres are hygroscopic fibres which upon the uptake of wound exudate become moist slippery or gelatinous and thus reduce the tendency for the surrounding fibres to adhere to the wound. The gel forming fibres are preferably spun sodium carboxymethylcellulose fibres, chemically modified cellulosic fibres, alkyl sulphonate modified cellulosic fibres such as those described in WO2012/061225, pectin fibres, alginate fibres, chitosan fibres, hyaluronic acid fibres, or other polysaccharide fibres or fibres derived from gums. The cellulosic fibres preferably have a degree of substitution of at least 0.05 carboxymethyl groups per glucose unit and more preferably are lightly substituted so that the absorbency of the fibres is limited. The gel forming fibres preferably have an absorbency of at least 2 grams 0.9% saline solution per gram of fibre (as measured by the method described above) but less than 30 grams 0.9% saline solution per gram of fibre. The gel forming fibres are preferably carboxymethylated cellulose fibres as described in PCT WO00/01425 to Azko Nobel UK Ltd which describes lightly carboxymethylated cellulose fabrics. The gel forming fibres are preferably lightly carboxymethylated in order to reduce the tendency of the absorbent layer to gel block and block the pathway for fluid from the wound, e.g. through the absorbent layer, the port and to a distal end of the conduit.

Preferably an absorbent layer is provided with fenestrations to aid the application of negative pressure to the wound and maintain the pathway for fluid from the wound, through the absorbent layer. Typically, however, fenestrations are only provided in internal absorbent layers.

The wound contact layer may be capable of absorbing exudate from the wound and transmitting it to the absorbent layer. Thus, there may be provided "internal" absorbent layers as defined above, preferably including fenestrations and an external absorbent layer, which forms the wound contact layer. Like the internal absorbent layer, the wound contact layer may be capable of allowing the passage of fluid through it so that pressure (either positive or negative) may applied to the wound and the pathway for fluid/exudate from the wound to the distal end of the conduit may be maintained.

The wound contact layer may include gel-forming fibres (e.g. of the type discussed herein), or a silicone gel, for example.

Preferably the wound contact layer comprises gel-forming fibres. The gel-forming fibres may be the same or a similar type to those comprising the absorbent layer but the wound contact layer may be strengthened to increase its integrity and that of the dressing. For example, the wound contact layer may be of the type described in EP 1904011 and comprise gel-forming fibres in the form of a mat with lines of longitudinal stitching made of cellulose or nylon or polyolefin yarn to increase the integrity of the layer. Preferably the wound contact layer is porous to maintain the pathway for fluid/exudate from the wound to the distal end of the conduit.

Preferably the one or more absorbent layer(s) comprise an internal absorbent layer provided with fenestrations to aid the application of negative pressure to the wound and maintain the pathway for fluid from the wound, through the internal absorbent layer and a wound contact layer comprising gel-forming fibres is also provided.

The (outer) covering layer of the dressing is provided as a bacterial and viral barrier layer which preferably resists the ingress of liquid and air but allows moisture vapour transmission. In this way the cover layer enhances the overall fluid handling capacity of the dressing by allowing for the escape of moisture vapour through the cover while enabling the application of pressure (either positive or negative) to the wound. The outer cover layer is for instance a layer having a MVTR of at least 10,000 g m⁻² per 24 hours or in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The cover layer may be in the form of a film of polyurethane, for example Epurex 912 T/129 manufactured by Covestro or Inspire 2350 manufactured by Coveris or Medifilm 426 manufactured by Mylan.

According to another aspect of the disclosure, not forming part of the invention as claimed, there is provided a connector part configured to connect a one-piece wound dressing selectively configurable for use in a pressure gradient wound therapy system and for use without a pressure gradient wound therapy system to a source of non-atmospheric pressure.

The connector part may form a first part of a two-part port; the wound dressing may comprise a second part of the two-part port; the second part of the two-part port may be a planar base plate.

According to another aspect of the disclosure, not forming part of the invention as claimed, there is provided a connector part of a two-part port configured to connect a one-piece wound dressing selectively configurable for use in a pressure gradient wound therapy system and for use without a pressure gradient wound therapy system to a source of non-atmospheric pressure; wherein the wound dressing comprises a second part of the two-part port; the second part of the two-part port being a planar base plate.

Of course, the one-piece wound dressing of this aspect of the invention may be the one-piece wound dressing of other aspects of the invention and have any of the optional features thereof.

The connector part may be injection moulded. The connector part may be flexible. The connector part may be comprised of thermoplastic. The connector part may be comprised of silicone.

The connector part may comprise a body. The body may be dome-shaped. The body may comprise a lower, (optionally planar) surface. The body may define a cavity having an opening in its lower (optionally planar) surface. The cavity may be hemispherical.

The connector part may comprise a tubing connection. The tubing connection may project from an edge of the body. The tubing connection may be configured to receive a tube connected to a source of non-atmospheric pressure.

The connector part may comprise a mechanical valve. The connector part may comprise a filter. The connector part may comprise a semipermeable membrane.

The cavity may be configured to connect to, for example to receive, the second part of the two-part port. The second part of the two part port may be the base plate as defined in the aspects above (optionally including any optional features). The base plate may be attached to the wound dressing. The opening in the body of the connector part may be configured, shaped and/or sized to match and/or align with an aperture in the base plate and optionally to an aperture in the wound dressing when the connector part and base plate of the two-part port are connected. The cavity comprise corresponding fastening means to the base plate. The cavity may be configured to receive projections projecting from the base plate. one or more recesses may be provided in the cavity to receive clips on the projections of the base plate. The one or more recesses may be circumferential. The cavity comprise a threaded inner wall. The cavity may comprise a slot or a pin of a bayonet connector.

The cavity may comprise a first opening of a conduit extending through the connector part body. A tubing connection may comprise a second opening of a conduit extending through the connector part body. The conduit may provide fluid, e.g. gaseous and/or liquid communication between the opening of the cavity and the tubing connection. The opening of the cavity may be orthogonal to the tubing connection.

The connector part may be fluidically coupled (e.g. by tubing) to a cannister. The cannister may be configured to collect excess exudate removed from the wound dressing.

According to another aspect of the invention there is provided a selectively configurable one-piece wound dressing comprising a base plate of a two-part port; and a connector part of a two-part port.

The selectively configurable one-piece wound dressing of this aspect may be the one-piece wound dressing of the other aspects of this invention and may have the optional features thereof.

Likewise the connector part of the wound dressing of this aspect may be the connector part of the other aspects of this invention and may have the optional features thereof.

According to another aspect of the disclosure, not forming part of the invention as claimed, there is provided a kit of parts including a selectively configurable one-piece wound dressing, the wound dressing being configurable for use in a pressure gradient wound therapy system and without a pressure gradient wound therapy system; the kit further comprising at least one of: (a) packaging indicating that the wound dressing is selectively configurable for use in a pressure gradient wound therapy system and without a pressure gradient wound therapy system; (b) instructions instructing a user as to how to configure the wound dressing for use in a pressure gradient wound therapy system and for use without a pressure gradient wound therapy system; (c) a piercing tool configured to pierce a covering layer of the wound dressing to produce an aperture therein and thereby configure the wound dressing for use in a pressure gradient wound therapy system; (d) a source of non-atmospheric pressure; (e) a connector part for connection to the base plate of the wound dressing to connect the wound dressing to a source of non-atmospheric pressure; or (f) tubing for connection between a wound dressing and a source of non-atmospheric pressure.

The one-piece wound dressing may comprise a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, configured to sealingly receive a connector part of the two-part port.

According to another aspect of the disclosure, not forming part of the invention as claimed, there is provided a kit of parts including a selectively configurable one-piece wound dressing, the wound dressing being configurable for use in a pressure gradient wound therapy system and without a pressure gradient wound therapy system; the kit further comprising at least one of: (a) packaging indicating that the wound dressing is selectively configurable for use in a pressure gradient wound therapy system and without a pressure gradient wound therapy system; (b) instructions instructing a user as to how to configure the wound dressing for use in a pressure gradient wound therapy system and for use without a pressure gradient wound therapy system; (c) a piercing tool configured to pierce a covering layer of the wound dressing to produce an aperture therein and thereby configure the wound dressing for use in a pressure gradient wound therapy system; (d) a source of non-atmospheric pressure; (e) a connector part for connection to the base plate of the wound dressing to connect the wound dressing to a source of non-atmospheric pressure; or (f) tubing for connection between a wound dressing and a source of non-atmospheric pressure; wherein the wound dressing comprises a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, configured to sealingly receive a connector part of the two-part port.

As noted above, the optional features set out above are equally applicable to all aspects (or embodiments) of the invention. For example, the selectively configurable one-piece wound dressing of the aspects of the invention concerning kit may be the selectively configurable wound dressing of the aspects outlined above including any of the optional features set out.

In one embodiment there is provided a kit of parts including a selectively configurable one-piece wound dressing, the wound dressing being configurable for use in a pressure gradient wound therapy system and without a pressure gradient wound therapy system; the kit further comprising: (a) packaging indicating that the wound dressing is selectively configurable for use in a pressure gradient wound therapy system and without a pressure gradient wound therapy system; wherein the wound dressing comprises a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, the base plate configured to sealingly receive a connector part of the two-part port.

In another embodiment there is provided a kit of parts including a selectively configurable one-piece wound dressing, the wound dressing being configurable for use in a pressure gradient wound therapy system and without a pressure gradient wound therapy system; the kit further comprising: (b) instructions instructing a user as to how to configure the wound dressing for use in a pressure gradient wound therapy system and for use without a pressure gradient wound therapy system; wherein the wound dressing comprises a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, the base plate configured to sealingly receive a connector part of the two-part port.

In another embodiment there is provided a kit of parts including a selectively configurable one-piece wound dressing, the wound dressing being configurable for use in a pressure gradient wound therapy system and without a pressure gradient wound therapy system; the kit further comprising: (c) a piercing tool configured to pierce a covering layer of the wound dressing to produce an aperture therein and thereby configure the wound dressing for use in a pressure gradient wound therapy system; wherein the wound dressing comprises a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, the base plate configured to sealingly receive a connector part of the two-part port.

In another embodiment, there is provided a kit of parts including a selectively configurable one-piece wound dressing, the wound dressing being configurable for use in a pressure gradient wound therapy system and without a pressure gradient wound therapy system; the kit further comprising: (d) a source of non-atmospheric pressure; wherein the wound dressing comprises a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, the base plate configured to sealingly receive a connector part of the two-part port.

In another embodiment, there is provided a kit of parts including a selectively configurable one-piece wound dressing, the wound dressing being configurable for use in a pressure gradient wound therapy system and without a pressure gradient wound therapy system; the kit further comprising: (e) a connector part for connection to a base plate of the wound dressing to connect the wound dressing to a source of non-atmospheric pressure; wherein the wound dressing comprises a wound contact layer and a covering layer, the covering layer having a first surface facing the wound contact layer and defining a wound dressing cavity, and a second surface; wherein the second surface of the covering layer comprises the planar base plate, the base plate configured to sealingly receive a connector part of the two-part port.

In another embodiment, there is provided a kit of parts including a selectively configurable wound dressing, the wound dressing being configurable for use in a pressure gradient wound therapy system and without a pressure gradient wound therapy system; the kit further comprising: (f) tubing for connection between a wound dressing and a source of non-atmospheric pressure; wherein the wound dressing comprises a wound contact layer and a covering layer, the covering layer having a first surface facing the wound contact layer and defining a wound dressing cavity, and a second surface; wherein the second surface of the covering layer comprises a planar base plate of a two-part port for connecting to a source of non-atmospheric pressure, the base plate configured to sealingly receive a connector part of the two-part port.

Of course the kit may comprise various combinations of features a to f; for example the kit may comprise at least two features including a+b; the kit may comprise at least two features including a+c; the kit may comprise at least two features including a+d; the kit may comprise at least two features including a+e; the kit may comprise at least two features including a+f; the kit may comprise at least two features including b+c; the kit may comprise at least two features including b+d; the kit may comprise at least two features including b+e; the kit may comprise at least two features including b+f; the kit may comprise at least two features including c+d; the kit may comprise at least two features including c+e; the kit may comprise at least two features including c+f; the kit may comprise at least two features including d+e; the kit may comprise at least two features including d+f; or the kit may comprise at least two features including or e+f. The kit may comprise at least three features including a+b+c; the kit may comprise at least three features including a+b+d; the kit may comprise at least three features including a+b+e; the kit may comprise at least three features including a+b+f; the kit may comprise at least three features including a+c+d; the kit may comprise at least three features including a+c+e; the kit may comprise at least three features including a+c+f; the kit may comprise at least three features including a+d+e; the kit may comprise at least three features including a+d+f; the kit may comprise at least three features including a+e+f; the kit may comprise at least three features including b+c+d; the kit may comprise at least three features including b+c+e; the kit may comprise at least three features including b+c+f; the kit may comprise at least three features including b+d+e; the kit may comprise at least three features including b+d+f; the kit may comprise at least three features including b+e+f; the kit may comprise at least three features including c+d+e; the kit may comprise at least three features including c+d+f; the kit may comprise at least three features including c+e+f; or the kit may comprise at least three features including d+e+f. The kit may comprise at least four features including a+b+c+d; the kit may comprise at least four features including a+b+c+e; the kit may comprise at least four features including a+b+c+f; the kit may comprise at least four features including b+c+d+e; the kit may comprise at least four features including b+c+d+f; the kit may comprise at least four features including b+d+e+f; or the kit may comprise at least four features including c+d+e+f. The kit may comprise at least five features including a+b+c+d+e; the kit may comprise at least five features including a+b+c+d+f; the kit may comprise at least five features including a+b+c+e+f; the kit may comprise at least five features including a+b+d+e+f; the kit may comprise at least five features including a+c+d+e+f; or the kit may comprise at least five features including b+c+d+e+f. The kit may comprise all six features; a+b+c+d+e+f.

One particular embodiment includes at least one item selected from a+b; one particular embodiment includes at least one item selected from a+c; one particular embodiment includes at least one item selected from a+d; one particular embodiment includes at least one item selected from a+e; one particular embodiment includes at least one item selected from a+f; one particular embodiment includes at least one item selected from b+c; one particular embodiment includes at least one item selected from b+d; one particular embodiment includes at least one item selected from b+e; one particular embodiment includes at least one item selected from b+f; one particular embodiment includes at least one item selected from c+d; one particular embodiment includes at least one item selected from c+e; one particular embodiment includes at least one item selected from c+f; one particular embodiment includes at least one item selected from d+e; one particular embodiment includes at least one item selected from d+f; one particular embodiment includes at least one item selected from e+f; one particular embodiment includes at least one item selected from a+b+c; one particular embodiment includes at least one item selected from a+b+d; one particular embodiment includes at least one item selected from a+b+e; one particular embodiment includes at least one item selected from a+b+f; one particular embodiment includes at least one item selected from a+c+d; one particular embodiment includes at least one item selected from a+c+e; one particular embodiment includes at least one item selected from a+c+f; one particular embodiment includes at least one item selected from a+d+e; one particular embodiment includes at least one item selected from a+d+f; one particular embodiment includes at least one item selected from a+e+f; one particular embodiment includes at least one item selected from b+c+d; one particular embodiment includes at least one item selected from b+c+e; one particular embodiment includes at least one item selected from b+c+f; one particular embodiment includes at least one item selected from b+d+e; one particular embodiment includes at least one item selected from b+d+f; one particular embodiment includes at least one item selected from b+e+f; one particular embodiment includes at least one item selected from c+d+e; one particular embodiment includes at least one item selected from c+d+f; one particular embodiment includes at least one item selected from c+e+f; one particular embodiment includes at least one item selected from d+e+f; one particular embodiment includes at least one item selected from a+b+c+d; one particular embodiment includes at least one item selected from a+b+c+e; one particular embodiment includes at least one item selected from a+b+c+f; one particular embodiment includes at least one item selected from b+c+d+e; one particular embodiment includes at least one item selected from b+c+d+f; one particular embodiment includes at least one item selected from b+d+e+f; one particular embodiment includes at least one item selected from c+d+e+f; one particular embodiment includes at least one item selected from a+b+c+d+e; one particular embodiment includes at least one item selected from a+b+c+d+f; one particular embodiment includes at least one item selected from a+b+c+e+f; one particular embodiment includes at least one item selected from a+b+d+e+f; one particular embodiment includes at least one item selected from a+c+d+e+f; one particular embodiment includes at least one item selected from b+c+d+e+f.

The packaging may for example be cardboard packaging. The packaging may comprise printed information indicating that the wound dressing is selectively configurable for use in a pressure gradient wound therapy system and without a pressure gradient wound therapy system. The packaging may be sterile; and/or at least part of the packaging, or a sub-package may be sterile.

The instructions instructing a user as to how to configure the wound dressing for use in a pressure gradient wound therapy system and for use without a pressure gradient wound therapy system may be printed on packaging. The instructions instructing a user as to how to configure the wound dressing for use in a pressure gradient wound therapy system and for use without a pressure gradient wound therapy system may be provided on an instruction sheet (which where the kit includes features a+b may be provided in the packaging).

The piercing tool configured to pierce a covering layer of the wound dressing to produce an aperture therein and thereby configure the wound dressing for use in a pressure gradient wound therapy system may comprise a blade. The piercing tool configured to pierce a covering layer of the wound dressing to produce an aperture therein and thereby configure the wound dressing for use in a pressure gradient wound therapy system may for example be scalpel.

The source of non-atmospheric pressure preferably a source of negative pressure. Alternatively, it may be a source of positive pressure. The source of non-atmospheric pressure may be a pump.

The connector part for connection to the base plate of the wound dressing to connect the wound dressing to a source of non-atmospheric pressure may be provided with an airway. The connector part may comprise an opening, at its distal end, for connecting the dressing to a source of pressure (either positive or negative), for example a pump. Preferably the connector is a Luer lock to facilitate secure connection to the pump and to maintain the pressure within the wound dressing while the pump is temporarily disconnected. The connector preferably comprises a one-way lock to assist in the maintenance of the applied pressure. To resist collapse, the airway may comprise an internal cylinder of nylon fibres to maintain openness of the airway to fluid.

The tubing for connection between a wound dressing and a source of non-atmospheric pressure may be transparent tubing. The tubing may be flexible. The tubing may be resilient. The tubing may be formed from a resilient flexible plastics material.

According to another aspect of the disclosure, not forming part of the invention as claimed, there is provided a pressure gradient wound therapy apparatus, comprising the kit of any preceding aspect of the invention.

In embodiments, the wound therapy apparatus comprises a negative pressure wound therapy apparatus. In other embodiments, the wound therapy apparatus comprises a positive pressure wound therapy apparatus.

In embodiments, the apparatus may comprise a canister and the wound dressing may be fluidly connected to the canister into which exudate removed from the wound may be withdrawn. In preferred embodiments, the wound dressing may be formed of a hydrocolloid material which may gel in the presence of an exudate and the apparatus may include no cannister. This may be referred to as a "canister-less" system.

The pump assembly may be fluidly connected to an interior region of the wound dressing, for introducing and/or removing gas from within the wound dressing to control the pressure therein.

According to a further broad aspect of the disclosure, not forming part of the invention as claimed, there is provided a method of configuring a selectively configurable one-piece wound dressing, the wound dressing being configurable for use in a pressure gradient wound therapy system and without a pressure gradient wound therapy system; the method comprising configuring the wound dressing for use with a pressure gradient wound therapy system by connecting a second part (i.e. a connecting part) of a two-part port to the wound dressing.

The wound dressing may comprise a covering layer, which may comprise a base plate of the two-part port. The method may comprise connecting the connecting part of the two-part port to the base plate.

According to an aspect of the disclosure, not forming part of the invention as claimed, there is provided a method of configuring a selectively configurable one-piece wound dressing, the wound dressing being configurable for use in a pressure gradient wound therapy system and without a pressure gradient wound therapy system; the wound dressing comprising a covering layer, which comprises a base plate of a two-part port; the method comprising configuring the wound dressing for use with a pressure gradient wound therapy system by connecting a connecting part of the two-part port to the base plate.

Of course, the one-piece wound dressing of the method aspects may be the one-piece wound dressing of the aspects of the wound dressing outlined above, or the wound dressing of the kit. And again, of course the wound dressing may comprise any optional feature described above.

The method may further comprise a step of piercing the covering layer to form an aperture. The aperture may be positioned to align with a hole in the base plate. The covering layer may be pierced with a surgical instrument (for example a scalpel).

The wound dressing may comprise a pressure distribution layer, in which case the method may comprise not cutting into the pressure distribution layer, or cutting into but not cutting through the pressure distribution layer. Similarly, the wound dressing may comprise a wound contact layer and the method may comprise not cutting into the wound contact layer, or cutting into, but not cutting through the wound contact layer. The wound dressing may comprise a pressure distribution layer and a wound contact layer and the method may comprise cutting through the covering layer, cutting into, but not through, the pressure distribution layer, and not cutting into the wound contact layer.

The wound dressing may comprise a release layer and the method may comprise applying a connecting part to the base plate, then removing the release layer, then applying the dressing to a wound.

The method may be a method of re-purposing a wound dressing that is provided on a wound and configured for use without a pressure gradient wound therapy system; the method comprising connecting a connector part to a base plate *in-situ* in order to configure the wound dressing for use with a pressure gradient wound therapy system.

The method may comprise attaching a non-atmospheric pressure source to the wound dressing after configuring the wound dressing for use with a pressure gradient wound therapy system.

### Detailed Description of the Invention

In order that the invention may be more clearly understood one or more embodiments thereof will now be described, by way of example only, with reference to the accompanying drawings, of which:
- Figure 1: is a schematic representation of an embodiment of a wound dressing configured for use without a wound therapy apparatus;
- Figure 2: is a schematic representation of the wound dressing of figure 1 configured for use with a wound therapy apparatus;
- Figure 3: is an isometric view of the unconnected port components of the wound dressings of figures 1 and 6;
- Figure 4: is an isometric view of the connected port components of the wound dressings of figures 2, 7 and 8;
- Figure 5: is a cross sectional view of the connected port components of figure 4;
- Figure 6: is an exploded view of a wound dressing according to another embodiment of the invention, configured for use without a wound therapy apparatus;
- Figure 7: is a cross sectional view through the wound dressing of figure 6, configured for use with a wound therapy apparatus;
- Figure 8: is a schematic representation of a wound exudate management system according to certain embodiments; and
- Figure 9: is a schematic representation of a kit of parts.

Embodiments disclosed herein relate to apparatus and methods (not forming part of the invention as claimed) of treating a wound both with and without reduced or positive pressure (typically negative pressure). Some embodiments including pump and wound dressing component. The wound dressings discussed are "one-piece" dressings incorporating both a covering layer and an absorbent body.

As disclosed herein the present invention may comprise a wound dressing; also disclosed but not forming part of the invention as claimed are a kit comprising the wound dressing and other apparatus for providing pressure gradient wound therapy to a wound.

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, or epidermolysis bullosa (EB).

Examples of other wounds include, but are not limited to, abdominal wounds or other large or incisional wounds (either as a result of surgery, trauma, stemiotomies, fasciotomies, or other conditions), dehisced wounds, acute wounds, chronic wounds, subacute and dehisced wounds, traumatic wounds (such as from orthopaedic trauma), flaps and skin grafts, lacerations, abrasions, contusions, burns, diabetic ulcers, pressure ulcers, stoma, surgical wounds, trauma and venous ulcers, broken bones or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

The technology disclosed can be used on an acute or chronic wound.

Wounds are believed to be more susceptible to infection under the following circumstances. If the wounds are chronic wounds, or if an object which caused the wound was dirty or contained bacteria, or from a bite, or contains remnant or a whole object that caused the wound, or a wound that is large or deep, or jagged edges to the wound, or elderly, or chronic because by their nature a wound site is open; and/or if the patient has: diabetes type 1 or type 2, is elderly, or has a compromised immune system.

Pressure gradient wound therapy may also be useful for treating second- and third-degree burns, as well as being useful for laparotomy surgery i.e., a large incision through an abdominal wall to gain access into the abdominal cavity.

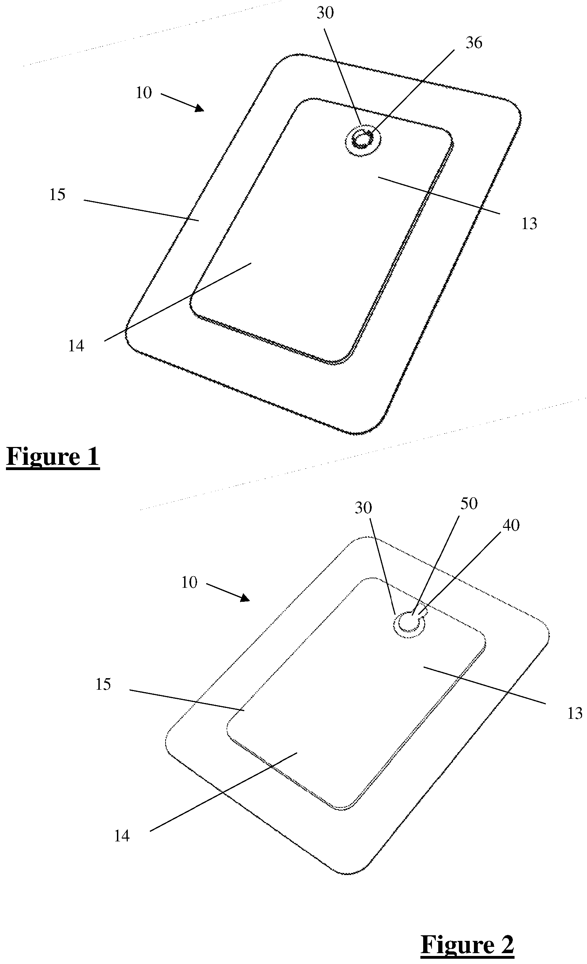

Figures 1 and 2 illustrate an embodiment of a wound dressing 10 in accordance with the invention in two different configurations.

In general, the disclosure relates to a wound dressing 10, which is selectively configurable for use without a pressure gradient wound therapy system or for use with a pressure gradient wound therapy system, e.g. negative pressure wound therapy and comprises a base plate 30 of a two-part port to enable connection of a source of non-atmospheric pressure for pressure gradient wound therapy, but can equally be used without a source of non-atmospheric pressure for "normal" wound therapy.

As shown in figure 1, the wound dressing 10 includes a covering layer 13 of the dressing 10. The covering layer 13 has a raised central region 14, where it overlies a dressing body, which can include a pressure dispersion layer; an absorbent/superabsorbent layer/layers; and a wound-contact layer. The dressing 10 also has a border region 15, where it overlies an adhesive layer. A removable release layer (not shown) is provided on the underside.

Arranged on the outer surface of the covering layer 13 is the base plate 30 (described in detail below); the base plate 30 is arranged towards the periphery of the raised central region 14.

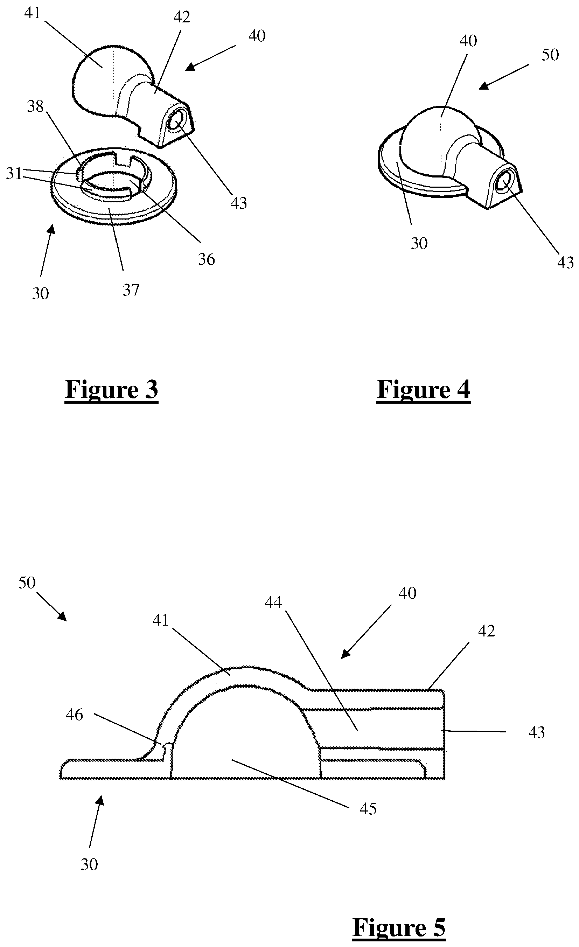

Figure 3 shows the base plate 30 and a connector part 40, which together make up a two-part port in an disassembled configuration. The connector base plate 30 is an annular, low profile thermoplastic plate having an aperture 36 in the center. The connector base plate 30 has a lower surface (not shown), which is adhered to the covering layer of wound dressing 30 (not shown in figure 3). Opposite the lower surface is an upper surface 37, the upper surface comprises three projections 38 arranged on an inner edge of the upper surface 37, the three projections 38 project perpendicular to the upper surface 37. The projections 38 are each provided with radially outwardly extending clips 31, which form a first part of a cantilever snap fitting, which is the exemplary means of connecting the connector base plate 30 and the connector part 40.

As shown in figures 3 to 5 the connector part 40 comprises a dome shaped body 41 and a tubing connection 42 projecting from the edge of the hemispherical body 41. A conduit 44 is provided within the second port component 40 extending between an opening 43 on the tube connecting portion 42 (for connection to tubing (not shown) and the open underside 45 of a hemispherical cavity beneath the dome 41 (as shown in figure 5). The inner edge of the hemispherical cavity is configured to provide the second part of the cantilever snap fitting, and as such includes a circumferential recess 46 configured to receive the clips 31 of the projections 38 of the first part of the cantilever snap fitting.

As illustrated in figure 1, to use the wound dressing 10 without a pressure gradient wound therapy system, the release layer (not shown) on the underside of the dressing 10 is simply removed and the dressing 10 applied in the same way as an ordinary wound dressing.

Figure 2 shows the wound dressing 10 configured for use with a pressure gradient wound therapy system. Here, the user has pierced the covering layer 13 in the region defined by the aperture 36 in the connector base plate 30 to form an aperture in the covering layer 13 and connected the connector part 40 to the base plate 30 by means for the snap fitting, so as to form an assembled port 50 (that is, the assembled port 50 comprises the connector base plate 30 and the second port component 40 as shown in figure 4). In this exemplary embodiment the user pierces the covering layer 13 with a scalpel (or alternatively scissors). When the user sealingly connects the base plate 30 to the connector part 40, such that the upper surface 37 of the base plate receives the underside of the connector part 40. The upper surface 37 of the base plate and underside of the connector part 40 form seal such that the assembled port 50 is configured to connect to a source of non-atmospheric, e.g. negative, pressure to the aperture without drawing in an excess of air at the interface of the base plate 30 and the connector part 40. The seal is preferably air tight, or at least substantially air-tight, so as to allow for the efficient supply of negative pressure to the wound dressing 10 to aid the wound-healing process. It will be appreciated by those skilled in the art that a completely air-tight seal is ideal, but with a sufficiently powerful pump, the seal need not be absolutely air-tight. In some embodiments the seal may be provided by the provision of a sealing element, such as an O-ring, arranged on one of the base plate or connector part. Those skilled in the art will appreciate that, as an alternative, the seal may be provided through the selection of materials and tolerances for the base plate 30 and connector part 40. As a consequence of the air tight nature of the seal between the base plate 30 and connector part 40, any excess extrudate being transferred from the wound dressing to a cannister will also be prevented from leaking from the assembled port.

The assembled port 50 can be connected via tubing (not shown in figure 2) to a pump (also not shown in figure 2) producing negative pressure. Those skilled in the art will appreciate that the steps to configure the wound dressing 10 for use with a pressure gradient wound therapy system can be undertaken either prior to or after the wound dressing 10 has been applied to the wound (i.e. the wound dressing can be configured for use with a pressure gradient wound therapy system *in-situ*)*.*

For use where it is envisioned that a wound will initially require treatment with a pressure gradient wound therapy system, the wound dressing 10 may be configured for use with a source of non-atmospheric pressure (as outlined above) prior to removing the release layer and applying the wound dressing 10 to the wound; as such the assembly force as the connector part 40 is applied to the base plate 30 can take place away from the wound, before the dressing is applied.

Then, if/when the pressure gradient therapy is no longer necessary, the user can use up any remaining wound dressings 10 without the pressure gradient wound therapy system, by configuring the wound dressing 10 for use without a source of non-atmospheric pressure (as outlined above, simply applying the dressing to the wound without connecting the connector part 40 to the base plate 30).

On the other hand, for use where it is envisioned that a wound does not require treatment with a pressure gradient wound therapy system (and most preferably where it is envisioned that a wound does not require treatment with a pressure gradient wound therapy system, but it is considered that there is a risk that the wound will not heal well without a pressure gradient so in future, pressure gradient wound therapy might be useful), the user can apply the wound dressing 10 to the wound in the configuration for use without a pressure gradient wound therapy system. That is, remove the release layer and apply the wound dressing to the wound, so that the wound contact layer 330 covers the wound without connecting the connector part 40 to the base plate 30.

Then, should it be determined that the wound would benefit from pressure gradient therapy (e.g. negative pressure), the user can configure the wound dressing 10 *in-situ,* that is, connect connector part 40 to the base plate 30 and apply a source of non-atmospheric pressure as outlined above, without having first to remove the dressing 10 (which can present an opportunity for infection).

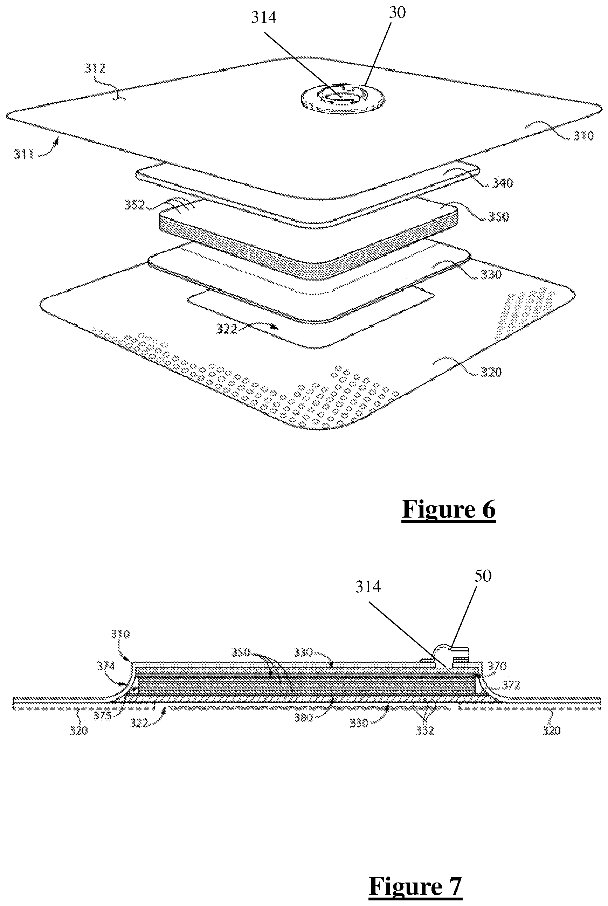

With reference to Figures 6 and 7, illustrated therein is a further exemplary embodiment of the present invention.

The illustrated wound dressing 300 generally includes a covering layer 310 and an adhesive layer 320 for adhering the wound dressing 300 adjacent the wound. In certain embodiments, the wound dressing 300 further comprises a wound contact layer 330 for contacting the wound, a pressure dispersion layer 340, a plurality of absorbent material layers 350 disposed between the wound contact layer 330 and the pressure dispersion layer 340.

The covering layer 310 has a first surface 311 and a second surface 312, and the first surface 311 is adjacent, and in contact with, the pressure dispersion layer 340 and the adhesive layer 320. The covering layer 310 defines a cavity in which the pressure dispersion layer 340 is arranged. In certain embodiments, the covering layer 310 is formed of a polyurethane film. The covering layer 310 comprises an aperture 314, providing fluid communication between the cavity and an environment external to the dressing. The aperture 314 arranged in the optimal position for connection to a source of negative pressure. The aperture 314 is surrounded by a base plate 30 of a two-part connector. The base plate 30 being of the same type as that described in the previous embodiment and being joined to the second surface 312 with an adhesive. In some embodiments, the aperture may be covered by a semi-permeable membrane, or a filter layer (not shown in figures 6 and 7). The semi-permeable membrane or filter layer provide a microbial barrier to restrict or prevent the ingress on bacterial and/or viruses to the wound when the dressing is used without a pressure therapy system.

The adhesive layer 320 generally defines a border about an opening 322 for receiving the wound. In certain embodiments, the adhesive layer 320 comprises a silicone adhesive. In certain embodiments, the adhesive layer 320 may be perforated.

The wound contact layer 330 overlaps the border defined by the adhesive layer 320, and is configured to contact the wound via the opening 322. In certain embodiments, the wound contact layer 330 may comprise Medicel^{™}. In certain embodiments, the wound contact layer 330 comprises carboxymethylated cellulose fibers. In certain embodiments, the wound contact layer 330 may comprise HYDROFIBER^{®}. In certain embodiments, the wound contact layer 330 may be reinforced, for example via nylon stitching. Thus, the wound contact layer 330 may comprise reinforcing nylon stitching 332.

The pressure dispersion layer 340 is adjacent and in contact with the first surface 311 of the cover layer 310. In certain embodiments, the pressure dispersion layer 340 may be provided as a polyester foam layer. In certain embodiments, the pressure dispersion layer 340 comprises reticulated foam.

The absorbent material layers 350 are positioned between the wound contact layer 330 and the pressure dispersion layer 340. The wound dressing 300 may, for example, comprise eight absorbent material layers 350. In certain embodiments, one or more of the absorbent material layers 350 may comprise carboxymethylated cellulose fibers. In certain embodiments, one or more of the absorbent material layers 350 may comprise Medicel^{™}. In certain embodiments, one or more of the absorbent material layers 350 may comprise HYDROFIBER^{®}. In certain embodiments, one or more of the absorbent material layers 350 further comprises fenestrations 352.

In certain embodiments, as shown in figure 7, the wound dressing 300 may include an additional layer 370 between the pressure dispersion layer 330 and the uppermost absorbent layer 350. The additional layer 370 may, for example, be formed of thermoplastic. In certain embodiments, the additional layer 370 may be provided as a thermoplastic spun lace layer. In certain embodiments, the wound dressing 300 may further comprise a nonwoven spun lace layer 372 connected to the wound contact layer 330. In certain embodiments, an envelope structure 374 is formed by joining peripheral portions 373 of the thermoplastic spun lace layer 370 and the nonwoven spun lace layer 372 such that the plurality of absorbent material layers 350 are disposed substantially within an interior cavity 375 of the envelope structure 374. In certain embodiments, the absorbent material layers 350 are disposed within the interior cavity 375 of the envelope structure 374.

In certain embodiments, the wound dressing 300 may include a further layer 380 positioned between the wound contact layer 330 and the lowermost absorbent layer 350. The further layer 380 may, for example, be a polyester/viscose layer.

As is well known, and therefore not shown, the wound dressing 300 may be provided with a removable release layer on the underside, covering the adhesive layer 320 and the underside of the wound contact layer 330; and it may be individually packaged within a sterile package.

As illustrated in figures 6, the wound dressing 300 is configured for use without a pressure gradient wound therapy system. To use the wound dressing 300 without a pressure gradient wound therapy system, it is simply removed from its sterile packaging, then the release layer is simply removed and the dressing applied in the same way as an ordinary wound dressing.

Figures 7 shows the wound dressing 300 of figures 6 configured for use with a pressure gradient wound therapy system. Here, a user has attached the connector part 40 of a two-part port to the base plate 30, by means of a snap fit attachment, to form an assembled port 50, the assembled port 50 of present invention being of the same type described in the previous embodiment.

It will be appreciated that the features of the wound dressing of either embodiment outlined above may be found in the wound dressing of the other embodiment. For example, the wound dressing 10 according to the first embodiment may have any or all of the layers of the wound dressing 300 according to the second embodiment; i.e. the wound dressing 10 may have one or more of the absorbent material layers as described, the wound dressing 10 may have one or more of the pressure distribution layers as described.

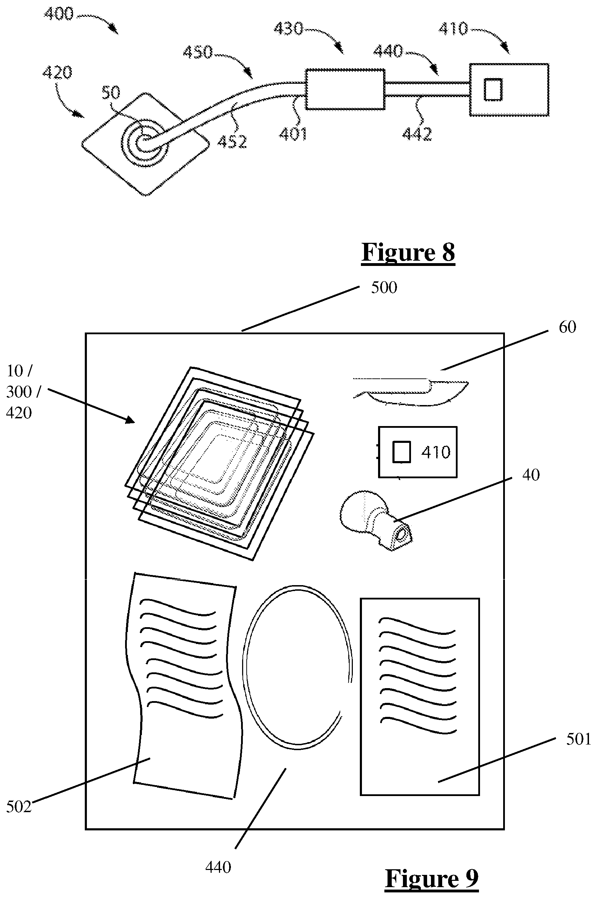

With additional reference to Fig. 8, illustrated therein is a pressure gradient wound therapy system 400 according to certain embodiments. The pressure gradient wound therapy system 400 comprises a pump 410 for generating negative pressure, a wound dressing 420 (which may be the wound dressing 10 or 300 described above) for covering and protecting a wound, an inline filter 430, a first pressure tube 440 having a first interior lumen 442, a second pressure tube 450 having a second interior lumen 452, and a two-part port 50. The first pressure tube 440 is disposed between the pump 410 and the inline filter 430. The second pressure tube 450 is disposed between the inline filter 430 and the port 50. The port 50 is disposed between the second pressure tube 450 and the wound dressing 420 such that the pump 410 and the wound dressing 420 are in fluid communication via the interior lumens 442, 452.

The wound dressings 10, 300, 420 may be provided in kits. As illustrated schematically in figure 9, the kits can comprise one or preferably more than one of the selectively configurable wound dressings 10, 300, 420 and at least one of the following items, all of which are included in this exemplary kit:
(a) packaging 500 indicating that the wound dressing is selectively configurable for use in a pressure gradient wound therapy system and without a pressure gradient wound therapy system. In this example the packaging is a cardboard box, printed with instructions 501 for use.
(b) instructions instructing a user as to how to configure the wound dressing for use in a pressure gradient wound therapy system and for use without a pressure gradient wound therapy system. In this example, the instructions are printed on a leaflet 502 included in the box.
(c) a piercing tool configured to pierce a covering layer of the wound dressing to produce an aperture therein and thereby configure the wound dressing for use in a pressure gradient wound therapy system. In this embodiment, the piercing tool is again a scalpel 60.
(d) a source of non-atmospheric pressure. In this embodiment, the source of non-atmospheric pressure is the pump 410.
(e) a connector part for connection to the base plate of the wound dressing to connect the wound dressing to a source of non-atmospheric pressure. In this embodiment the connector part is the connector part 40 described above.
(f) tubing for connection between a wound dressing and a source of non-atmospheric pressure. In this embodiment tubing 440 described above is included.

For use where it is envisioned that a wound will initially require treatment with a pressure gradient wound therapy system, the wound dressing 10 may be configured for use with a source of non-atmospheric pressure (as outlined above) prior to removing the release layer and applying the wound dressing 10 to the wound. The kit may comprise one or more preferably a plurality of, dressings 10/300/420 and one or more of items c, d, and e, for example all of items c, d and e, and optionally items a and/or b as well. As such, the user can be provided with all the equipment required to use the wound dressings 10/300/420 with a pressure gradient wound therapy system.

In order to use such a kit, the user (e.g. a patient or HCP) can cut an aperture in a dressing 10/300/420 in the region within the aperture 36 in the base plate 30; attach a connector part 40 thereto, around the aperture, and in fluid communication therewith; attach one end of the tubing 440 to the port 50 and the other to the pump 410 and run the pump 440 to provide non-atmospheric (e.g. negative) pressure to the wound.

Then, if/when the pressure gradient therapy is no longer necessary, the user can use up any remaining wound dressings 10 without the pressure gradient wound therapy system, by configuring the wound dressing 10 for use without a source of non-atmospheric pressure (as outlined above).

On the other hand, for use where it is envisioned that a wound does not require treatment with a pressure gradient wound therapy system (and most preferably where it is envisioned that a wound does not require treatment with a pressure gradient wound therapy system, but it is considered that there is a risk that the wound will not heal well without a pressure gradient so in future, pressure gradient wound therapy might be useful), the kit may comprise one, or more preferably a plurality of selectively configurable dressings 10/300/420 and one or both of items a and b. As such, the user has instructions on how to use the dressings without a pressure gradient wound therapy system and can simply apply a dressing 10/300/420 to the wound in the configuration for use without a pressure gradient wound therapy system. Thus the user can follow the instructions by removing the release layer and applying the dressing to the wound without connecting the connecting part 40 of the two-part port.

Then, should it be determined that the wound would benefit from pressure gradient therapy (e.g. negative pressure), the user can configure the wound dressing *10*/*300*/*420 in-situ,* that is, follow the instructions from the packaging/instructions, to (optionally cut a hole in the covering layer 3 through the aperture 36 if using a wound dressing 10 according to he first embodiment; and) apply a connector part 40 of the two-part port to the base plate 30 and the other items of a pressure gradient wound therapy system as outlined above (sourced for example from another kit), without having to first remove the dressing 1/300/420 (which can present an opportunity for infection).

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment. Except in Examples, or where otherwise explicitly indicated, all numerical quantities in this description specifying amounts of materials, device dimension, and the like, are to be understood as modified by the word "about."

Unless otherwise indicated, each chemical or composition referred to herein should be interpreted as being a commercial grade material which may contain the isomers, by-products, derivatives, and other such materials which are normally understood to be present in the commercial grade.

The one or more embodiments are described above by way of example only. Many variations are possible without departing from the scope of protection afforded by the appended claims.

## Claims

1. A one-piece wound dressing (10,300,420) selectively configurable for use in a pressure gradient wound therapy system and for use without a pressure gradient wound therapy system; the wound dressing (10,300,420) comprising;
a wound contact layer (330) having a first surface for contacting a wound and an opposing second surface; and
a covering layer (13,310) having a first surface (311) facing the wound contact layer and defining a wound dressing cavity, and an opposing second surface (312);
wherein the second surface (312) of the covering layer comprises a planar base plate (30) of a two-part port (50) for connecting to a source of non-atmospheric pressure and configured to sealingly receive a connector part (40) of the two-part port,
wherein a body of the wound dressing comprises the wound contact layer (330), and the covering layer (13,310) and the body of the wound dressing are provided as an integral item **characterised in that** the base plate (30) comprises a microbial barrier.

2. A wound dressing (10,300,420) according to claim 1 wherein the integral item includes an adhesive layer (320).

3. A wound dressing (10,300,420) according to claim 1 or 2 wherein the integral item includes a removeable release layer.

4. A wound dressing (10,300,420) according to any preceding claim wherein the base plate (30) is flexible.

5. A wound dressing (10,300,420) according to any preceding claim wherein the microbial barrier is a mechanical valve.

6. A wound dressing (10,300,420) according to any preceding claim wherein the covering layer (13,310) comprises an aperture (314), the base plate (30) surrounding the aperture (314), and wherein the aperture (314) is covered by the microbial barrier.

7. A wound dressing (10,300,420) according to any preceding claim wherein a pressure distribution layer (340) is provided adjacent to the covering layer (13,310).

8. A wound dressing (10,300,420) according to any preceding claim wherein the adhesive layer (320) is provided on the underside of the covering layer (13,310) so as to form an adhesive border.

9. A wound dressing (10,300,420) according to any preceding claims further comprising one or more absorbent layers (350) in combination with a gel-forming fibre.

10. A wound dressing (10,300,420) according to any preceding claim wherein the base plate (30) comprises one or more projections (38) for connecting the connector part (40) to the base plate, wherein the projections (38) are arranged adjacent the aperture (314).

11. A wound dressing (10,300,420) according to claim 10 wherein the projections (38) are configured to connect to the connector part (40) by means of a snap fit.

12. A wound dressing (10,300,420) according to any preceding claim wherein the base plate comprises a sealing element.

13. A selectively configurable wound dressing (10,300,420) comprising a base plate (30) of a two-part port (50) according to any preceding claim; and a connector part (40) of a two-part port.

14. A wound dressing (10,300,420) according to claim 13 wherein the connector part (40) comprises a tubing connection, the tubing connection (42) being configured to receive a tube connected to a source of non-atmospheric pressure.

15. A wound dressing (10,300,420) according to claim 14 wherein the connector part comprises a mechanical valve.

## Patentansprüche

1. Einteiliger Wundverband (10,300,420), selektiv konfigurierbar zur Verwendung in einem Druckgradienten-Wundtherapiesystem und zur Verwendung ohne ein Druckgradienten-Wundtherapiesystem; der Wundverband (10,300,420) aufweisend;
eine Wundkontaktschicht (330) mit einer ersten Oberfläche zum Kontaktieren einer Wunde und einer gegenüberliegenden zweiten Oberfläche; und
eine Abdeckschicht (13,310) mit einer ersten Oberfläche (311), die der Wundkontaktschicht zugewandt ist und einen Wundverbandhohlraum definiert, und einer gegenüberliegenden zweiten Oberfläche (312);
wobei die zweite Oberfläche (312) der Abdeckschicht eine planare Grundplatte (30) eines zweiteiligen Ports (50) zum Verbinden mit einer Quelle von nicht-atmosphärischem Druck aufweist und konfiguriert ist, ein Verbindungsteil (40) des zweiteiligen Ports dichtend aufzunehmen,
wobei ein Körper des Wundverbands die Wundkontaktschicht (330) und die Abdeckschicht (13,310) aufweist und der Körper des Wundverbands als ein integraler Gegenstand bereitgestellt ist, **dadurch gekennzeichnet, dass** die Grundplatte (30) eine mikrobielle Barriere aufweist.

2. Wundverband (10,300,420) nach Anspruch 1, wobei der integrale Gegenstand eine Haftschicht (320) enthält.

3. Wundverband (10,300,420) nach Anspruch 1 oder 2, wobei der integrale Gegenstand eine entfernbare Freigabeschicht enthält.

4. Wundverband (10,300,420) nach einem der vorhergehenden Ansprüche, wobei die Grundplatte (30) flexibel ist.

5. Wundverband (10,300,420) nach einem der vorhergehenden Ansprüche, wobei die mikrobielle Barriere ein mechanisches Ventil ist.

6. Wundverband (10,300,420) nach einem der vorhergehenden Ansprüche, wobei die Abdeckschicht (13,310) eine Öffnung (314) aufweist, wobei die Grundplatte (30) die Öffnung (314) umgibt, und wobei die Öffnung (314) durch die mikrobielle Barriere abgedeckt ist.

7. Wundverband (10,300,420) nach einem der vorhergehenden Ansprüche, wobei eine Druckverteilungsschicht (340) angrenzend zur Abdeckschicht (13,310) bereitgestellt ist.

8. Wundverband (10,300,420) nach einem der vorhergehenden Ansprüche, wobei die Haftschicht (320) auf der Unterseite der Abdeckschicht (13,310) bereitgestellt ist, um einen haftenden Rand zu bilden.

9. Wundverband (10,300,420) nach einem der vorhergehenden Ansprüche, ferner aufweisend eine oder mehrere absorbierende Schichten (350) in Kombination mit einer gelbildenden Faser.

10. Wundverband (10,300,420) nach einem der vorhergehenden Ansprüche, wobei die Grundplatte (30) einen oder mehrere Vorsprünge (38) zum Verbinden des Verbindungsteils (40) mit der Grundplatte aufweist, wobei die Vorsprünge (38) angrenzend zur Öffnung (314) angeordnet sind.

11. Wundverband (10,300,420) nach Anspruch 10, wobei die Vorsprünge (38) konfiguriert sind um sich mittels einer Schnappverbindung mit dem Verbindungsteil (40) zu verbinden.

12. Wundverband (10,300,420) nach einem der vorhergehenden Ansprüche, wobei die Grundplatte ein Dichtelement aufweist.

13. Selektiv konfigurierbarer Wundverband (10,300,420), aufweisend eine Grundplatte (30) eines zweiteiligen Ports (50) nach einem der vorhergehenden Ansprüche; und ein Verbindungsteil (40) eines zweiteiligen Ports.

14. Wundverband (10,300,420) nach Anspruch 13, wobei das Verbindungsteil (40) einen Schlauchanschluss aufweist, wobei der Schlauchanschluss (42) konfiguriert ist, einen Schlauch aufzunehmen, der mit einer Quelle von nicht-atmosphärischem Druck verbunden ist.

15. Wundverband (10,300,420) nach Anspruch 14, wobei das Verbindungsteil ein mechanisches Ventil aufweist.

## Revendications

1. Un pansement (10, 300, 420) d'une seule pièce configurable sélectivement pour utilisation dans un système de thérapie de plaie à gradient de pression et pour utilisation dans un système de thérapie de plaie sans gradient de pression ; le pansement (10, 300, 420) pour plaie comprenant :
une couche (330) de contact avec la plaie ayant une première surface pour entrer en contact avec une plaie et une deuxième surface opposée ; et
une couche (13, 310) de recouvrement ayant une première surface (311) faisant face à la couche de contact avec la plaie et définissant une cavité de pansement pour plaie et une deuxième surface (312) opposée ;
dans lequel la deuxième surface (312) de la couche de recouvrement comprend une plaque (30) de base plane d'un orifice (50) en deux parties pour communiquer avec une source de pression non atmosphérique et configurée pour recevoir avec étanchéité une partie (40) de connecteur de l'orifice en deux parties,
dans lequel un corps du pansement pour plaie comprend la couche (330) de contact avec la plaie et la couche (13, 310) de recouvrement et le corps du pansement pour plaie sont prévus sous la forme d'un article d'un seul tenant, **caractérisé en ce que** la plaque (30) de base comprend une barrière microbienne.

2. Un pansement (10, 300, 420) pour plaie suivant la revendication 1, dans lequel l'article d'un seul tenant comprend une couche (320) adhésive.

3. Un pansement (10, 300, 420) pour plaie suivant la revendication 1 ou 2, dans lequel l'article d'un seul tenant comprend une couche amovible de libération.

4. Un pansement (10, 300, 420) pour plaie suivant l'une quelconque des revendications précédentes, dans lequel la plaque (30) de base est souple.

5. Un pansement (10, 300, 420) pour plaie suivant l'une quelconque des revendications précédentes, dans lequel la barrière microbienne est une soupape mécanique.

6. Un pansement (10, 300, 420) pour plaie suivant l'une quelconque des revendications précédentes, dans lequel la couche (13, 310) de recouvrement comprend une ouverture (314), la plaque (30) de base entourant l'ouverture (314), et dans lequel l'ouverture (314) est recouverte de la barrière microbienne.

7. Un pansement (10, 300, 420) pour plaie suivant l'une quelconque des revendications précédentes, dans lequel une couche (340) de répartition de la pression est prévue au voisinage de la couche (13, 310) de recouvrement.

8. Un pansement (10, 300, 420) pour plaie suivant l'une quelconque des revendications précédentes, dans lequel la couche (320) adhésive est prévue du côté en dessous de la couche (13, 310) de recouvrement, de manière à former une bordure adhésive.

9. Un pansement (10, 300, 420) pour plaie suivant l'une quelconque des revendications précédentes, comprenant en outre une ou plusieurs couches (350) absorbantes en combinaison avec une fibre géliforme.

10. Un pansement (10, 300, 420) pour plaie suivant l'une quelconque des revendications précédentes, dans lequel la plaque (30) de base comprend une ou plusieurs saillies (38) de liaison de la partie (40) de connecteur à la plaque de base, dans lequel les saillies (38) sont disposées au voisinage de l'ouverture (314).

11. Un pansement (10, 300, 240) pour plaie suivant la revendication 10, dans lequel les saillies (38) sont configurées pour relier la partie (40) de connecteur au moyen d'un ajustement à déclic.

12. Un pansement (10, 300, 420) pour plaie suivant l'une quelconque des revendications précédentes, dans lequel la plaque de base comprend un élément d'étanchéité.

13. Un pansement (10, 300, 420) pour plaie configurable sélectivement comprenant une plaque (30) de base d'un orifice (50) en deux parties suivant l'une quelconque des revendications précédentes ; et une partie (40) de connecteur de l'orifice en deux parties.

14. Un pansement (10, 300, 420) pour plaie suivant la revendication 13, dans lequel la partie (40) de connecteur comprend une connexion tubulaire, la connexion (42) tubulaire étant configurée pour recevoir un tube communiquant avec une source de pression non atmosphérique.

15. Un pansement (10, 300, 420) pour plaie suivant la revendication 14, dans lequel la partie de connecteur comprend une soupape mécanique.
